# EUROPEAN PATENT APPLICATION

(11) **EP 3 461 832 A1**
(43) Date of publication of application: **03.04.2019**
(21) Application number: 17194093.5
(22) Date of filing: 29.09.2017
(51) Int. Cl.: C07H 1/00, C07H 21/02, C07H 21/04, C12Q 1/6844, C12Q 1/6855, C12Q 1/686, C12Q 1/6874

(54) **CLICK BASED LIGATION**

(71) Applicant: baseclick GmbH, 82061 Neuried (DE)
(72) Inventor: FRISCHMUTH, Thomas, 12247 Berlin (DE); SERDJUKOW, Sascha, 83088 Kiefersfelden (DE)
(74) Representative: Weickmann & Weickmann PartmbB

(57) **Abstract**

The present invention relates to new methods and reagents for coupling molecules by a so-called click reaction in the presence of a suitable catalyst and divalent metal cations. Further, the invention relates to an activator composition for such click ligation reaction, a click ligation reagent kit, a device for performing such click ligation reaction, and the use of such method, composition, reagent kit and device to improve the efficiency of coupling of molecules via a click reaction, especially in the context of next generation nucleic acid sequencing methods.

SEQUENCE LISTING

<110>  baseclick GmbH

<120>  Click based ligation

<130>  64516P EP

<140>  EP 17 194 093.5
<141>  2017-09-29

<160>  12

<170>  PatentIn version 3.5

<210>  1
<211>  59
<212>  DNA
<213>  Artificial Sequence

<220>
<223>  Alkyne oligo1

<220>
<221>  modified_base
<222>  (1)..(1)
<223>  5'-alkyne dT

<400>  1
taatgatacg gcgaccaccg agatctacac tctttcccta cacgacgctc ttccgatct        59

<210>  2
<211>  59
<212>  DNA
<213>  Artificial Sequence

<220>
<223>  Azide oligo1

<220>
<221>  modified_base
<222>  (1)..(1)
<223>  5'-azide dT

<400>  2
tggagttcgt gaccgccgcc gggatcactc tcggcatgga cgagctgtac aagtaaagc        59

<210>  3
<211>  58
<212>  RNA
<213>  Artificial Sequence

<220>
<223>  model RNA

<400>  3
uucgacaaac gaaaacacaa acacaaacca aacagaaaac aguacaugua aucgacca        58

<210>  4
<211>  17
<212>  DNA
<213>  Artificial Sequence

<220>
<223>  reverse transcription Primer1


<220>
<221>  modified_base
<222>  (1)..(1)
<223>  5'-FAM-dT
(FAM=Fluorescein)

<400>  4
tggtcgatta catgtac                                                    17


<210>  5
<211>  23
<212>  DNA
<213>  Artificial Sequence

<220>
<223>  reverse trascription Primer2

<400>  5
tggtcgatta catgtactgt ttt                                             23


<210>  6
<211>  17
<212>  DNA
<213>  Artificial Sequence

<220>
<223>  reverse transcription Primer3

<400>  6
agatcggaag agcgtcg                                                    17


<210>  7
<211>  57
<212>  DNA
<213>  Artificial Sequence

<220>
<223>  cDNA after reverse transcription


<220>
<221>  modified_base
<222>  (1)..(1)
<223>  5'-FAM-dT
(FAM=Fluorescein)

<220>
<221>  modified_base
<222>  (57)..(57)
<223>  3'-azide dA

<400>  7
tggtcgatta catgtactgt tttctgtttg gtttgtgttt gtgttttcgt ttgtcga          57


<210>  8
<211>  116
<212>  DNA
<213>  Artificial Sequence

<220>
<223>  click product


<220>
<221>  modified_base
<222>  (1)..(1)
<223>  5'-FAM dT
(FAM=Fluorescein)

<220>
<221>  misc_feature
<222>  (62)..(63)
<223>  A and T joined via backbone mimic B

<400>  8
tggtcgatta catgtactgt tttctgtttg gtttgtgttt gtgttttcgt ttgtcgataa       60

tgatacggcg accaccgaga tctacactct ttccctacac gacgctcttc cgatct          116


<210>  9
<211>  116
<212>  DNA
<213>  Artificial Sequence

<220>
<223>  PCR product

<400>  9
tggtcgatta catgtactgt tttctgtttg gtttgtgttt gtgttttcgt ttgtcgataa       60

tgatacggcg accaccgaga tctacactct ttccctacac gacgctcttc cgatct          116


<210>  10
<211>  51
<212>  DNA
<213>  Artificial Sequence

<220>
<223>  Fig.6 expected sequence

<400>  10
gttttctgtt tggtttgtgt ttgtgttttc gtttgtcgat aatgatacgg c               51


<210>  11
<211>  51
<212>  DNA
<213>  Artificial Sequence

<220>
<223>  Fig.6 found sequence

<400>  11
gttttctgtt tggtttgtgt ttgtgttttc gtttgtcgat aatgatacgg c                51

<210>  12
<211>  21
<212>  DNA
<213>  Artificial Sequence

<220>
<223>  Fig.6 forward seq avg. QV=43

<400>  12
gtttgtcgat aatgatacgg c                                                 21

## Description

The present invention relates to new methods and reagents for coupling molecules by a so-called click reaction in the presence of a suitable catalyst. Further, the invention relates to an activator composition for such click ligation reaction, a click ligation reagent kit, a device for performing such click ligation reaction, and the use of such method, composition, reagent kit and device to improve the efficiency of coupling of molecules via a click reaction, especially in the context of next generation nucleic acid sequencing methods.

### Background of the invention

In 2001/2002 the groups of Sharpless and Meldal independently defined the concept of "Click chemistry" and the criteria for a transformation to be considered as a "Click" reaction (Sharpless, K.B. et al, Angew. Chem 2002, 114, 2708; Angew. Chem. Int. Ed. 2002, 41, 2596, Meldal, M. et al, J. Org. Chem., 2002, 67, 3057). Since then, the copper catalyzed reaction of azides with alkynes to give 1,2,3-triazoles, a variation of the 1,3-dipolar Huisgen cycloaddition (R. Huisgen, 1,3-Dipolar Cycloaddition Chemistry (Ed.: A. Padwa), Wiley, New York, 1984) has become the most widely used Click reaction. As a result of its mild conditions and high efficiency, this reaction has found a myriad of applications in biology and material sciences, such as e. g. DNA labeling purposes (Gramlich, P.M.A. et al., Postsynthetic DNA Modification through the Copper-Catalyzed Azide-Alkyne Cycloaddition Reaction. Angew. Chem. Int. Ed. 2008, 47, 8350).

The rapid analysis of genetic material for a specific target sequences, e.g. for the presence of single nucleotide polymorphism, the presence of a certain gene like a resistance gene, or of mRNA requires easy to use, efficient and reliable tools. A major problem is the need to detect DNA or RNA of interest directly in small biological samples such as patient blood or plants. These provide the analyte only in minute amounts. In order to reach the required sensitivity, an amplification step is usually required wherein either the nucleic acid analyte is amplified prior to analysis or a detection method is used in which the minute detection signal, directly obtained from the DNA/RNA analyte, is amplified.

Methods for the amplification of the nucleic acid analyte include PCR and other nucleic acid amplification protocols. PCR amplification has the major advantage that, within a pool of different DNA strands obtained from a biological material, only the DNA sequence of interest is amplified. This is the basis for a reliable analysis of single genes in complex biological samples.

Enzymatic ligation of oligonucleotides is a standard procedure in numerous protocols for oligonucleotide manipulation and is required for sequencing, cloning and many other DNA- and RNA-based technologies. The enzymes involved in the catalysis of the ligation reaction form phosphodiester bonds between 5'-phosphate ends of DNA or RNA and 3'-hydroxyl ends. In order to work efficiently, the ligases require double-stranded oligonucleotides, which are preferably pre-organized by using cohesive ends or even splint strands. The ligation reaction can join any 5'-phosphate with any 3'-hydroxyl end, since the reaction is not sequence specific. This lack of substrate specificity is a major advantage for a broad general application of enzymatic ligations and has contributed to its wide application. Ironically, this is at the same time an immense challenge for many sequencing applications as sequence artifacts are generated from unspecific joining of oligonucleotide fragments. As almost every sequencing technology requires ligation of a known oligonucleotide sequence, the so-called adapters, for primer binding during sequencing and PCR amplification, this can generate artifactual recombination, sequence chimera formation (DNA-RNA strands for RNA sequencing) and adapter dimers (a pair of ligated adapters with no insert sequence) formation.

In order to reduce sequence artifacts, the 3'-end of the primer is blocked by insertion of a 2',3' dideoxynucleotide (ddNTP). This avoids adapter dimers formation and ensures correct directional ligation, but it cannot influence artifactual recombination and chimera formation. These effects need to be corrected using special algorithms during contig (a set of overlapping sequence data reads) generation from sequence reads.

The present invention is concerned with a click chemistry-based method for the non-enzymatic joining of molecules, especially oligonucleotides, which is efficient, site-specific and is compatible with PCR amplification. The reaction between two non-templated single-stranded oligonucleotides is more efficient than the comparable enzymatic ligation reaction, thus e.g. eliminating the need for second strand synthesis in e.g. RNA sequencing.

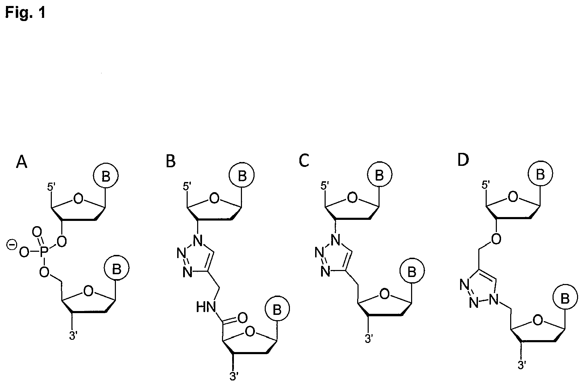

The preparation of alkyne- or azide-containing oligonucleotides has been studied extensively. Of special interest for the present invention are "backbone mimics", i.e. non-natural alternatives for the phosphodiester bond, which can be generated by copper-catalyzed azide alkyne cycloadditions (CuACC). Some of the resulting triazole-containing oligonucleotides can be converted into natural phosphodiester backbones by polymerase enzymes without mutation of the sequence and thus are fully biocompatible (Shivalingam et al., Molecular Requirements of High-Fidelity Replication-Competent DNA Backbones for Orthogonal Chemical Ligation (2017) doi: 10.1021/jacs.6b11530). Figure 1 shows the structure of some triazole backbone mimics that are generated by click chemistry, the natural phosphodiester is shown on the left side for comparison.

An especially useful application of this artificial DNA backbone technology lies in sample preparation for next-generation sequencing. Routh et al. (ClickSeq: Fragmentation-Free Next-Generation Sequencing via Click Ligation of Adaptors to Stochastically Terminated 3'-Azido cDNAs. J. Mol. Biol. 427, 2610-2616 (2015)) provide a protocol for RNA sequencing, in which an adapter oligonucleotide is clicked to a 3'-azide terminated cDNA fragment and a PCR reaction is performed to provide a cDNA library. In comparison with a standard library preparation protocol that involves enzymatic ligation of the adapter oligonucleotide, this method can drastically decrease the rate of artifactual recombination and sequence chimera formation.

A severe limitation to this application of the click technology in DNA or RNA sequencing so far is a less than satisfactory efficiency in which the 3'-azido-blocked cDNA fragments used in the method are captured during library generation. Only about 10% of the 3'-azido-blocked cDNA fragments could be ligated to an alkyne-modified oligonucleotide. Furthermore, the conversion of this triazole-linked single-stranded DNA into a double stranded DNA via read-through of the triazole linkage is not efficient. While for standard RNAseq experiments, such efficiency might be sufficient, for other applications requiring a thorough capture of the original RNA or DNA, the efficiency of the click reaction and the subsequent read-through is considered inadequate and must be improved.

Accordingly, it is an object of the present invention to provide means to achieve a higher efficiency especially of the click ligation in order to establish successful application of the click technology to the next generation sequencing.

### Summary of the invention

The present invention relates to improving the efficiency of ligating two reaction partners in a click reaction using an alkyne group and an azide group as the click functional groups. The click reaction as hereinafter intended is a reaction between a 1,3-dipolar moiety, which is an azide, with an unsaturated moiety, which is a terminal alkyne, catalyzed by a metal catalyst, especially a heterogeneous Cu(I) or another suitable metal catalyst. The click reaction in the catalyzed form is a non-concerted ionic mechanism which results in a 5-membered heterocyclic 1,2,3-triazole moiety.

According to a first aspect, the present invention relates to a method for coupling a first molecule to a second molecule in a click reaction, wherein the first molecule comprises a first click functional group which is an alkyne group, and the second molecule comprises a second click functional group, which is an azide group, the method comprising contacting the first and second molecules in a reaction mixture in the presence of a catalyst, preferably heterogeneous Cu catalyst under conditions wherein a click reaction between the first and second molecules occurs, said click reaction being a formal (3+2) cyclo-addition reaction forming a 1,2,3-triazole ring, the method being characterized in that the click reaction is performed in the presence of divalent metal cations in the reaction mixture.

A further aspect of the present invention is an activator composition for use in a click reaction, wherein a first molecule comprising a first click functional group, which is an alkyne group, and a second molecule comprising a second click functional group, which is an azide group, are coupled in the presence of a catalyst, preferably a heterogeneous Cu catalyst, said activator mixture comprising divalent metal cations, Cu(I)-stabilizing ligands and optionally an organic solvent.

Still a further aspect of the present invention is a click ligation reagent kit comprising at least as one component a catalyst and as a second component an activator composition according to the present invention.

Still a further aspect of the present invention relates to a device having at least one reaction chamber, wherein the reaction chamber comprises a catalyst, preferably a heterogeneous Cu catalyst for carrying out a click reaction between two reaction partners and further wherein divalent metal cations or an activator composition of the present invention are contained in the reaction chamber.

Still a further aspect of the present invention relates to the use of the above methods, compositions, reagent kits and devices for efficiently ligating molecules, preferably oligonucleotides via a click reaction. An example of such molecules carries a detectable label. The ligation can thus provide labeled biomolecules which may be used for detecting an analyte, e.g. a nucleic acid in a sample, particularly involving the use of a compound labeled by a click reaction, which forms an association product with the analyte to be detected. In particular, the methods, compositions, reagent kits and devices can be used in next generation sequencing technologies, including RNA library preparation, DNA library preparation, and in the analysis of complex oligonucleotide mixtures, especially parallel and multiplex RT-qPCR applications. Further, the methods, compositions, reagent kits and devices of the present invention can be used for sequencing by ligation methods (e.g. "Solid Sequencing" from ABI), in ligase chain reactions, in cloning methods, in particular to form a recombinant DNA, and in gene synthesis.

The research that led to the present invention has shown that surprisingly addition of divalent metal ions to a click reaction which is catalyzed by a catalyst, in particular by a Cu(I) catalyst, especially a heterogeneous Cu (I) catalyst, can vastly improve the oligonucleotide-oligonucleotide click reaction kinetics. This improvement allows efficient click reactions between the backbone functionalized oligonucleotides in yields that were until now only obtained by preorganization of the reaction partners through splint oligonucleotides.

### Detailed Description of Preferred Embodiments

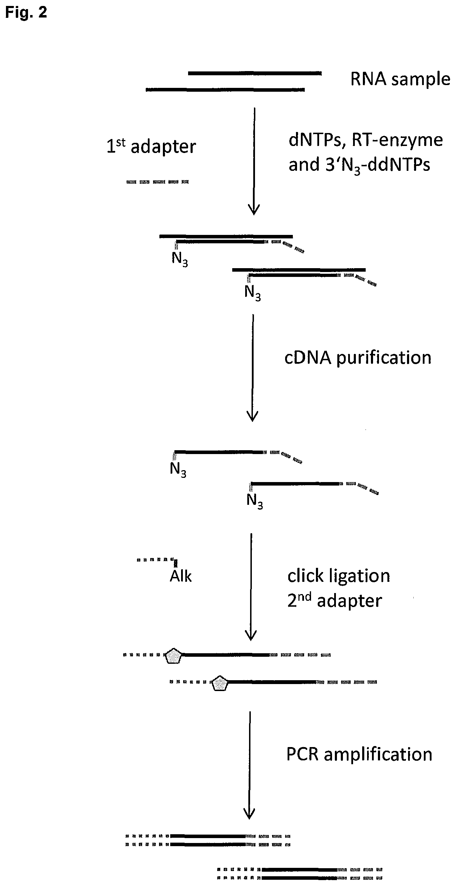

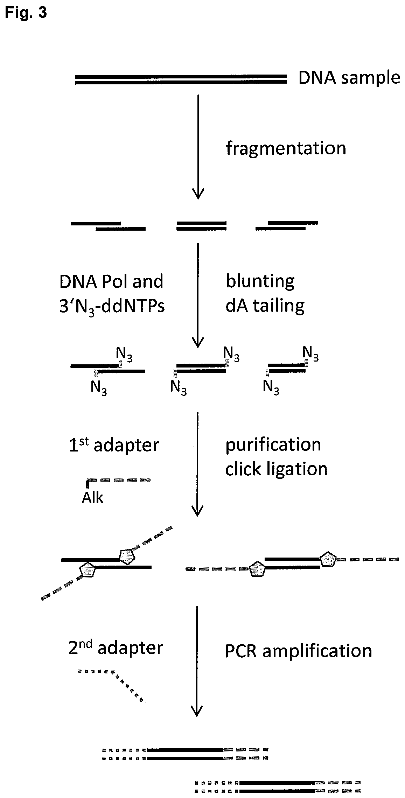

The present invention provides for improved conditions for click reactions, which avoid the need for splint oligonucleotides and still lead to high yields of the click ligation reaction. In an example of such click reaction which can be performed under the conditions of the present invention, a 3'-alkyne- or a 3'-azide-modified nucleotide, can be introduced into an oligonucleotide, e.g. by enzymatic incorporation during reverse transcription (RNA sequencing) or during blunting (single stranded DNA overhangs are filled with nucleotides and/or removed) and/or by dA tailing (non-templated addition of nucleotides, most often dATP, to the 3' end of blunt double-stranded DNA). For RNA sequencing, a first adapter (adapters are short oligonucleotides containing complementary sequences for primer binding and hybridization) can be introduced using a partly randomized primer. A second adapter is click ligated to the 3'-alkyne or 3'-azide terminated cDNA. A schematic presentation of such reaction during ClickSeq RNA library preparation is shown in Fig. 2. For DNA sequencing the first adapter is click ligated to the enzymatically 3'-alkyne or 3'-azide modified oligonucleotide (the second adapter is introduced during PCR via a partly complementary binding region (e. g. 12 mer) of the adapters). The respective ClickSeq DNA library preparation workflow is shown in Fig. 3. The primer or the adapter can contain an index sequence to allow for a subsequent correlation to one of different samples. This allows for applying the click sequencing technology to mixtures of several enzymatically produced samples. After removal of excess modified nucleotide, a click ligation is performed between the enzymatically produced and purified modified oligonucleotides and a 5'-alkyne- or 5'-azide modified oligonucleotide acting as an adapter in order to introduce nucleotide sequences that are necessary for PCR amplification and/or sequencing. To this end, the adapter oligonucleotide preferably is produced via solid phase synthesis and the 5'-modification introduced at the end of such synthesis.

As the alkyne modification element, commercially available building blocks can be used, e.g. having the following structure:

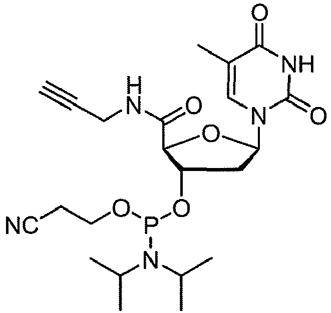

As a catalyst for the click reaction, a Cu catalyst, preferably a heterogeneous Cu(I)-catalyst is used. In particular, a catalyst as described in EP 2 416 878 B1 can serve as the source for the catalytic active copper species for the click reaction.
In the present invention, the click reaction comprises a copper catalyzed variation of a formal (3+2) cycloaddition between the azide and the terminal alkyne group. The irreversible formation of 1,2,3-triazoles as a result of the azide/alkyne reaction is biorthogonal due to the lack of azides and terminal alkynes in organisms, the required chemical groups are small (incorporation with minimal disruption of the biomolecule's environment) and the reaction is regioselective, resulting in exclusive formation of the 1,4 regioisomer.

The following reaction is the basis of the click ligation of azides and terminal alkynes 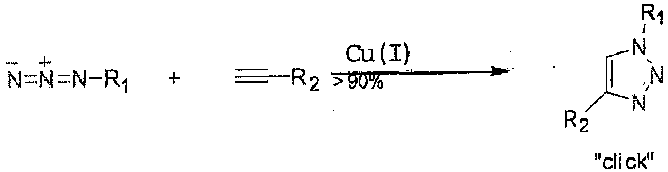 wherein R₁ and R₂ are first and second partners in the click reaction.

The catalyst is most preferably a heterogeneous Cu-catalyst, more preferably a heterogeneous Cu(I)-catalyst. It should be noted, however, that other metal catalysts and especially other heterogeneous metal catalysts, such as Zr, W, Fe, Ru, Co, Th, Ir; Ni, Pd, Pt, Ag, Au, Zn, Cd, Hg and other metal ions have been reported to contribute directly or indirectly to a catalysis of the click reaction ligation and can also be used within the context of the present invention. Alternatively, homogenous Cu(I)-catalyst can be used for the click ligation. The heterogeneous Cu catalyst is a metal-C-catalyst or elemental copper, i.e. a solid Cu catalyst comprising a carbon-based support such as charcoal having incorporated Cu ions therein or elemental copper, which can generate Cu(I) ions. In an especially preferred embodiment, the heterogeneous Cu catalyst is a Cu(I)-C-catalyst which may be prepared as described by H.Lipshutz and B.R.Taft in Angew. Chem. Int. Ed., 2006, 45, 8235-8238.

The heterogeneous catalyst may be a particulate catalyst, e.g. a heterogeneous catalyst consisting of particles having a size of from 10 nm to 1000 µm, preferably from 100 µm to 800 µm. Alternatively, the catalyst may also be a porous non-particulate catalyst, e.g. a solid matrix having embedded therein catalytically active particles.

In a further embodiment, a further solid carrier material is included which is a material different from the heterogeneous Cu catalyst, e.g. a chromatographic material on which a biomolecule such as preferably a nucleic acid or a nucleic acid analog can be immobilized. Preferably, materials are included which allow for a separation and purification of the click reaction product from the other constituents of the reaction mixture. Exemplary mechanisms for separation and purification are size exclusion or affinity chromatography.

Examples of suitable chromatographic materials are an ion exchange material, a hydrophilic material or a hydrophobic material. In a preferred embodiment a hydrophilic material, e.g. silica gel, can be used in combination with the heterogeneous catalyst. In another preferred embodiment a hydrophobic material, e.g. silica C18 or C4 or an ion exchange resin, can be used in combination with the heterogeneous catalyst. In a still further preferred embodiment, the solid carrier material may be a resin which is used for the solid phase synthesis of biomolecules, especially nucleic acids and nucleic acid analogs.

It was found that the click reaction between an immobilized reaction partner and a reaction partner which is freely present in solution may be effectively catalyzed by a heterogeneous Cu catalyst system. This strategy may allow achieving simultaneously the click reaction and the purification and/or the separation of the product from the impurities and/or from excess reagents or salts eventually present in the reaction mixture.

In the method according to the present invention, the reaction mixture contains divalent metal cations. Preferred divalent metal cations are earth alkaline metals, or other divalent metal ions with similar properties like e.g. divalent manganese (Mn²⁺) or zinc (Zn²⁺). In an especially preferred embodiment of the present invention, Mg²⁺ ions are present in the reaction mixture, which have been shown to be especially suitable cations for the anionic DNA phosphate backbone. For this purpose, Mg²⁺ salts are included in the reaction mixture. The addition of the divalent metal cation and especially Mg²⁺ to the reaction mixture causes all possible ligand position for copper species on the phosphate backbone to be occupied by the divalent cations. As a consequence, the amount of copper species which is available as a catalyst in the reaction mixture is increased and the reaction kinetics is remarkably improved.

Suitable amounts of divalent metal cations in the click reaction mixture are 1 to 200 mmol/l, preferably 5 to 25 mmol/l, especially preferably 10 to 20 mmol/l.

In the click reaction according to the present invention, it is preferred to further include a Cu(I) stabilizing ligand. As such ligand, metal ligands, like amines and especially Tris(3-hydroxypropyltriazolylmethyl)amine (THPTA), 2-(4-((bis((1-(tert-butyl)-1H-1,2,3,-triazol-4-yl)methyl)amino)methyl)-1H-1,2,3-triazolyl-1-yl)acetic acid (BTTAA) or analogs thereof can be used in the reaction mixture. These metal ligands stabilize the Cu(I) catalyst and protect the oligonucleotides against formation of reactive oxygen species. Further, addition of such metal ligands also improves the reaction kinetics of the click reaction.

Metal ligands are included in the click reaction mixture in an amount of 10 to 4000 µmol/l, preferably 500 to 1000 µmol/l and especially preferably 700 to 900 µmol/l.

In a further preferred embodiment of the present invention, an organic solvent is added to the reaction mixture. In particular, dimethyl sulfoxide (DMSO) can be advantageously included in the reaction mixture. Addition of such organic solvent and especially DMSO has a further positive effect on the efficiency of the click reaction between two oligonucleotides. It is assumed that DMSO disturbs the secondary structures of oligonucleotides and thus improves the accessibility of the functional groups of the reaction partners, e.g. the azide and alkyne groups. Within the context of the present invention, addition to a final content of 1 to 10 % of such organic solvent, especially DMSO, to the reaction mixture is considered useful, addition of organic solvents to a final content of 2 to 8 %, and especially 4 to 6% in the reaction mixture is preferred.

Surprisingly it was observed that especially the addition of the divalent metal ions into the reaction mixture of the click reaction causes a remarkable increase in click reaction product formation. Compared with the reaction performed without addition of such divalent metal cations, the yield of click product could be at least doubled or even tripled. Using the reaction mixture which further contains the organic solvent, especially DMSO, and a Cu-stabilizing ligand further improves the performance and leads to highly satisfactory results and yields of click product. These effects of the method according to the present invention carry forward to subsequent reactions and uses of such click products, especially to PCR reactions for DNA or RNA library preparation and next generation sequencing. Thus, the object of the present invention could be solved with surprisingly simple means and the shortcomings of the method that are mentioned in Routh *et al.* be resolved accordingly.

Considering the relevance of divalent metal ions and some other substances for the efficiency of the click ligation, a further subject matter of the present invention is an activator composition for use in the click reaction for coupling molecules that are functionalized by on the one hand a terminal alkyne group and on the other hand an azide group in a Cu-catalyzed reaction, preferably a reaction carried out in the presence of a heterogeneous Cu catalyst. Such activator composition contains divalent metal cations. As mentioned above, addition of such divalent metal cations, preferably earth alkaline metal cations and especially preferably Mg²⁺ ions, prevents binding of the copper catalyst species to the DNA or RNA phosphate backbone. Such binding occurs without addition of such metal cations and reduces the amount of catalyst available for the click reaction. Due to the presence of divalent metal cations and the blocking of the binding sites on the phosphate backbone, the amount of copper that is available as a catalyst is increased and the reaction kinetics improved.

As also already explained above in detail, the presence of Cu(I)-stabilizing ligands and/or the presence of an organic solvent, especially DMSO further improves the efficiency of the click reaction between the two oligonucleotides. Accordingly, a preferred activator composition according to the present invention contains the divalent metal cations and an organic solvent and at least one Cu-stabilizing ligand. Especially preferred is an activator composition containing as the divalent metal cation Mg²⁺ and DMSO and at least one Cu-stabilizing ligand selected from THPTA, BTTAA, analogs thereof or any mixtures of such ligands.

The activator composition contains these effector molecules in amounts which provide for a concentration in the click reaction mixture of the divalent metal cations of 1 to 200 mmol/l, preferably 5 to 25 mmol/l and especially preferably 10 to 20 mmol/l; the Cu-stabilizing ligand of 10 to 4000 µmol/l, preferably 500 to 1000 µmol/l and especially preferably 700 to 900 µmol/l; and/or the organic solvent, preferably DMSO of 1 to 10%, preferably 2 to 8% and especially preferably 4 to 6%.

The activator composition according to the present invention can be provided as a composition containing one, two or all of the above-mentioned substances that help to improve the efficiency of the click reaction. The activator composition can be an aqueous composition containing a pre-dilution of the effector substances. The activator composition can also contain other solvents, especially further organic solvents or a combination of water and organic solvents. The activator composition can further contain other substances like buffer substances or any other substance that can be included in the performance of the click reaction.

A further subject matter of the present invention is a click ligation reagent kit, such reagent kit comprising at least as one component a catalyst, especially a Cu catalyst and preferably a heterogeneous Cu(I) catalyst, and as a second component an activator composition according to the present invention. As disclosed above in more detail, the activator composition has a distinct positive effect on the copper catalyzed click reaction in ligating alkyne-and azide-functionalized oligonucleotides. Accordingly, a reagent kit of the present invention provides the catalyst as well as the effector molecule(s) of the activator composition together in order to facilitate the implementation of the click ligation method disclosed herein. In preferred embodiments, the click ligation reagent kit contains as the activator composition the correspondingly preferred embodiment, wherein not only divalent metal cations, preferably earth alkaline metal cations and especially Mg²⁺, but also at least one of an organic solvent and a Cu-stabilizing ligand is present. For best results, an activator composition containing all three effector substances is included in the click ligation reagent kit of the present invention.

The Cu(I) catalyst that is included in the inventive click ligation reagent kit preferably is a heterogeneous catalyst as described in EP 2 416 878 B1.

Optionally, such reagent kit contains further substances that are required components and/or advantageous in performing a click reaction. In addition to the above-mentioned components, a click ligation reagent kit of the present invention can further contain at least one azide- or alkyne-functionalized oligonucleotide, which can act as an adapter or primer. The reagent kit can contain labels, labeled click functional groups, modified nucleotides, enzymes, buffers, primer, further carrier materials or chromatographic materials for purification, preferably materials as described above in the context of the click ligation method according to the invention.

In a further preferred embodiment of the present invention, the click ligation reagent kit can additionally contain substances and components required for a subsequent PCR reaction to amplify the click ligated product, for DNA or RNA library preparation and sequencing, e.g. for analyzing complex oligonucleotide mixtures.

A still further subject matter of the present invention is a device which facilitates performing the click reaction of the present invention. Such device comprises at least one reaction chamber, such reaction chamber comprising a catalyst, preferably a heterogeneous Cu catalyst for carrying out the click reaction between two reaction partners. Respective devices are disclosed and further described in EP 2 416 878 B1 and such disclosure applies also to devices which can be used according to the present invention.

According to the present invention, in addition to the presence of one or more components of a click ligation reaction, the device further comprises divalent metal cations, preferably earth alkaline metal cations and especially Mg²⁺, or an activator composition as described above within the one or more reaction chambers of such device. Since the click reaction may be performed in very small volumes, the devices can be e.g. microtiter plate wells, pipette tips or spin columns, comprising one or more compartments which can act as the at least one reaction chamber. Metal ions or other preferred components employed according to the present invention are present in such devices in the at least one of the reaction chambers where the click reaction is performed.

It is to be understood that within the context of the present invention all different manners of performing such click reaction and all substances that are described for such purposes are included as long as the essential requirements of the methods, compositions, reagent kits and devices of the invention as described above are met. Accordingly, all other forms, variations and improvements of click reactions which are not explicitly described herein are considered applicable also within the context of the present invention as long as the click reaction mixture for the catalyzed click ligation includes the divalent metal cations which have been shown by the present invention to have a considerable advantageous effect on the efficiency and yield of the click reaction. While the presence of these cations, especially earth alkaline cations and most preferably Mg²⁺ ions is mandatory for solving the object of the present invention, other aspects of the click reaction can vary dependent on e.g. the nature of the molecules to be coupled by click ligation, the actually used catalyst, the reaction conditions and the intended uses and respective subsequent reactions to be performed.

The use of the methods, compositions, reagent kits and devices for efficiently ligating molecules, preferably oligonucleotides, via click reaction in order to provide click reaction products which can be used in subsequent reactions is a further subject matter of the present invention. As such subsequent reactions, all reactions are included that require ligation products which have a high purity and are provided in a sufficient lead high yield to perform such subsequent reaction. Examples of such subsequent reactions are PCR or other amplification methods for analyzing DNA or RNA samples, especially where complex oligonucleotide mixtures are to be analyzed. The present invention allows to obtain click ligation products of oligonucleotides constituting "backbone mimics", i.e. of RNA or DNA molecules containing non-natural alternatives for phosphodiester bonds in their backbone. As it was found that such substances are fully biocompatible, the amplification of such molecules is highly effective and useful especially for the so-called next generation sequencing applications.

When the above stated conditions are applied e.g. to RNA sequencing library preparation, the second adapter can be efficiently click ligated to single stranded cDNA, thus making second strand synthesis needless and the total preparation time is reduced. Due to the bio-orthogonality and specificity of the click reaction, the click ligation occurs exclusively between azide- and alkyne-functionalized molecules. The azide is introduced stochastically during cDNA synthesis as the natural deoxynucleotides are supplemented with low concentration of e.g. 3'-azido-2',3'-dideoxynucleotides, which terminates the fragments. The 5'-alkyne modified adapter oligo can be prepared by solid-phase DNA synthesis and can be used without purification, since the 5'-alkyne is incorporated last in solid phase synthesis and shorter non-labeled strands cannot be click ligated.

The inventive click concept can especially preferably be applied to DNA library preparation protocols. The DNA fragments are generally modified by blunting and dA tailing through DNA polymerase to allow more efficient subsequent enzymatic ligation. Replacing the natural dNTPs with e.g. 3'-azido-2',3'-dideoxynucleotides during blunting or dA tailing introduces azide groups for click ligation to 5'-alkyne adapter oligonucleotides.
Application of the present invention in the context of the ClickSeq technology as described for example by Routh *et al*., *vide supra,* is also extremely valuable if complex oligonucleotide mixtures are to be analyzed. Parallel analyses for RNA-virus RNA can be done by performing multiplex RT-qPCR. For this purpose, several virus RNA specific primers (e.g. HIV, hepatitis C, etc.) are added to RNA in a sample for ClickSeq reverse transcription. Azide-terminated cDNA fragments are click ligated to an adapter to provide primer binding sites for subsequent PCR amplification. Due to the specificity of the click ligation, no artifactual recombination can occur, rendering separate qPCR setups for each detection unnecessary. Thus, the technology provides a basis for massive parallel RT-qPCR applications.

Application areas of this technology include all other DNA and RNA library preparation kits for sequencing, sequencing by ligation methods (e.g. "Solid Sequencing" from ABI), ligase chain reaction, cloning methods (to perform recombinant DNA) and gene synthesis, wherein two or more molecules are ligated via click functional groups. While partly in general and partly in more detail, possible applications have been described above, it is to be understood that other reactions including a click ligation performed under the conditions described herein are also included within the scope of the present invention.

### Description of the Figures

Fig. 1 shows the structure of some triazole backbone mimics generated by click chemistry (B-D) compared to the natural phosphodiester (A). Subsequent experimental examples in the context of PCR amplification are shown for the triazole backbone mimic (B) only;
Fig. 2 shows a schematic ClickSeq RNA library preparation which is one of the methods of interest in the present invention. It involves a combined cDNA synthesis, fragmentation and adapter click ligation. Second strand synthesis is obsolete since ssDNA can be efficiently click ligated;
Fig. 3 shows a schematic ClickSeq DNA library preparation workflow which is a further method of interest in the present invention. A sample double-stranded (ds) DNA is fragmented and the fragments are manipulated by blunting and dA tailing like in a standard DNA library preparation. By using e.g. 3'-azido-2',3'-dideoxynucleotides instead of natural dNTPs, 3'azide terminated dsDNA is obtained. After removal of excess nucleotides by fragment purification (including size selection), the first adapter is clicked to the fragment via its 5'-alkyne group. The second adapter is introduced during PCR amplification via a short (about 12 bp) 3'-sequence which is the reverse complement of the 5'-end of the first adapter and acts as the first primer for amplification;
Fig. 4 shows the influence of various cations on the click reaction efficiency and yield;
Fig. 5 shows PCR products of cDNA produced from a 3'-azide terminated cDNA and a 5'-alkyne adapter using different DNA polymerases; and
Fig. 6 shows the result of Sanger sequencing of the amplified click ligated product of Fig. 5;
Fig. 7 shows exemplary structures of azide and alkyne modified nucleotides for enzymatic incorporation and subsequent use in click ligation;
Fig. 8 shows structures of exemplary 5'-ends of adapter oligonucleotides for click ligation (A-C). 5'-alkyne modified oligonucleotide (with the base B = thymine of structure A) was used for the examples 1, 2 and 4 (in Figs. 4, 5 and 9). Structure B was used in the subsequent examples 1 and 4 (in Figs. 4 and 9).
Fig. 9 shows the oligo-oligo click reaction yield for a low oligo concentration.

The following examples are provided for illustration purposes.

### Example 1: Preparation of click products

In a 200 µL reaction vial a single reactor pellet (600-800 µm, containing elemental copper) was combined with 12.5 µL reaction mix and incubated at 45 °C for 60 min. The reaction mix consisted of 4 mM THPTA, 55 µM of an alkyne oligo1, 55 µM of an azide oligo1 and, when cation influence was studied 16 mM monovalent cations (or 8 mM divalent cation). dH₂O was used to adjust the volume to a final 12.5 µL if necessary.

After the incubation the sample was briefly spinned down and the supernatant was transferred to a new vial to stop the reaction. Samples were analyzed on 2.5% agarose gels (10 x 15 cm) prepared in TAE buffer (20 mM TRIS, 10 mM acetic acid, 0.5 mM EDTA).

Samples were prepared with 20% purple loading dye (NEB, New England BioLabs Inc.), and low molecular weight DNA ladder (25-766 bp, NEB, N3233) was prepared accordingly; usually 0.5 µL marker were used in 5 µL loading volume. Gels were run in TAE buffer applying constant power (10 W, max. 500 V, max. 100 mA) for 60 min. Then, gels were incubated in a freshly prepared 1:10 000 ethidium bromide dilution for 15 min and then destained in dH₂O for 15 min. For visualization a Gel Doc EZ Imager (Bio Rad) was used.

### Oligonucleotides

Alkyne oligo1: 
5'-TAA TGA TAC GGC GAC CAC CGA GAT CTA CAC TCT TTC CCT ACA CGA
CGC TCT TCC GAT CT-3'
T = 5'-alkyne dT 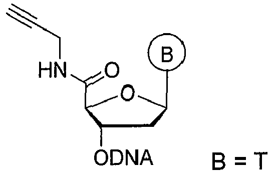
Azide oligo1:

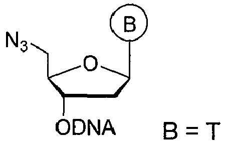

5'-N3-TGG AGT TCG TGA CCG CCG CCG GGA TCA CTC TCG GCA TGG ACG
AGC TGT ACA AGT AAA GC -3'

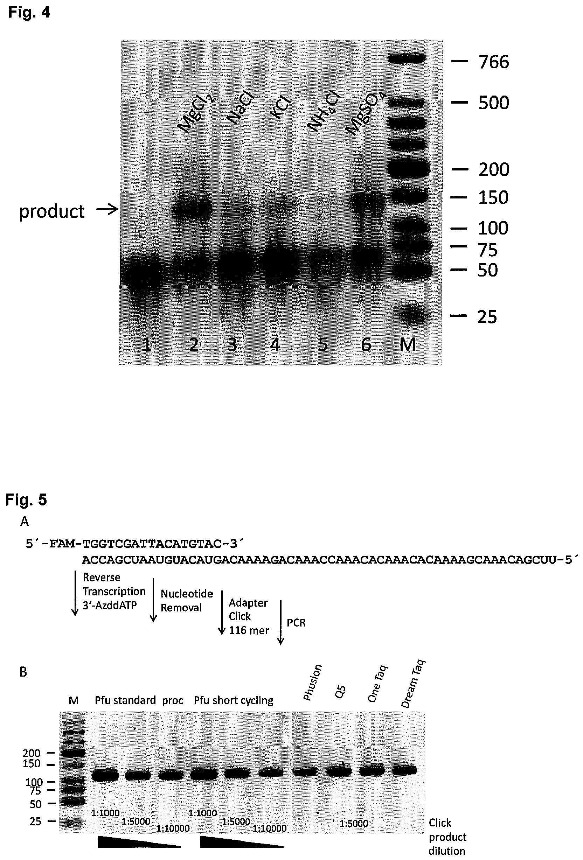

Due to the non-ideal click conditions used in this experiment (too much THPTA, not enough copper source), the influence of cation addition of (earth) alkaline metal addition becomes apparent. Figure 4 shows the 2.5% gel of the oligonucleotide click reaction and the influence of different cations on the click efficiency and product yield. In the absence of an additional cation (slot 1) a yield of less than 5% of the click product was observed under the conditions described above. Through addition of Mg²⁺ ions (8 mM) the yield is improved to about 30%. As a comparison, a concentration of 16 mM of monovalent cations was also analyzed, however only a slight improvement of the yield was observed (slots 3-5, yields of 5 to 10%).

### Example 2: PCR amplification of the click product

The feasibility of the ClickSeq protocol is exemplified for a model RNA sequence. The RNA was hybridized to primer1 and then reverse transcribed in the presence of 200 µM dTTP, dGTP, dCTP and 3'-azido-ddATP using MuLV reverse transcriptase. Nucleotides and enzyme were removed by purification of the cDNA using the nucleotide removal kit (Qiagen) according to manufacturers' instructions.

Alkyne oligo1 was clicked to the purified cDNA in a 200 µL reaction vial with a single reactor pellet (600-800 µm, containing elemental copper) in a total 12.5 µL reaction mix and incubated at 45 °C for 60 min.

The reaction mix consisted of 800 µM THPTA, 20 mM MgCl₂, 5% DMSO, 7 µM of alkyne oligo1 and about 4 µM purified cDNA. dH₂O was used to adjust the volume to a final 12.5 µL if necessary.

After the incubation the sample was briefly spinned down and the supernatant was transferred to a new vial to stop the reaction. The crude click reaction was diluted 1:1000, 1:5000 and 1:10000 (max. 4 nM, 0.8 nM and 0.4 nM) for PCR amplification without further purification.

In a 200 µL reaction vial, PCR amplifications were prepared in a total volume of 20 µL. Click reaction dilutions were combined with 200 µM dNTPs, 10 pmol of primer2 and primer3 and one unit polymerase. For the various polymerases, Pfu, Phusion, Q5, One Taq and Dream Taq buffers were used according to manufacturers' recommendations. The samples were subjected to a thermal cycling program in a thermocycler (BioRad).

As a standard cycling condition following conditions were used:

| **step** | **temperature** | **duration** | |
|---|---|---|---|
| 1 | 95 °C | 2 min | |
| 2 | 95 °C | 15 s | 25 x |
| 3 | 51 °C | 20 s | |
| 4 | 72 °C | 30 s | |
| 5 | 72 °C | 2 min | |

For the Pfu polymerase different template dilutions and an alternative cycling condition were studied:

| **step** | **temperature** | **duration** | |
|---|---|---|---|
| 1 | 95 °C | 2 min | |
| 2 | 95 °C | 15 s | 25 x |
| 3 | 52 °C | 5 s | |
| 4 | 72 °C | 20 s | |
| 5 | 72 °C | 2 min | |

After the incubation the sample was briefly spinned down and an aliquot was analyzed on 3% agarose gels (10 x 15 cm) prepared in TAE buffer (20 mM TRIS, 10 mM acetic acid, 0.5 mM EDTA).

Samples were prepared with 20% purple loading dye (NEB), and low molecular weight DNA ladder (25-766 bp, NEB, N3233) was prepared accordingly; usually 0.5 µL marker were used in 5 µL loading volume. Gels were run in TAE buffer applying constant power (10 W, max. 500 V, max. 100 mA) for 60 min. Then, gels were incubated in a freshly prepared 1:10 000 ethidium bromide dilution for 15 min and then destained in dH₂O for 15 min. For visualization a Gel Doc EZ Imager (Bio Rad) was used.

Figure 5A illustrates the ClickSeq workflow (described above) which was done for the shown model RNA. The workflow involves reverse transcription of the RNA into cDNA. By replacing natural dATP by 3'-AzddATP the cDNA is terminated with a 3'-azide. After removal of excess nucleotides by purification of the cDNA, a 5'-alkyne adapter is click ligated to the 3'-azide and the crude reaction mix is used as template for PCR.

Figure 5B is an ethidium bromide stained agarose gel of PCR samples treated according to the ClickSeq workflow for the model RNA in 5A. Since the triazole-containing template is amplified by various polymerases under different conditions, this illustrates the biocompatibility of the unnatural backbone mimic.

### Oligonucleotides

Alkyne oligo1 (see example 1)
Model RNA: 
5'-UUC GAC AAA CGA AAA CAC AAA CAC AAA CCA AAC AGA AAA CAG UAC
AUG UAA UCG ACC A-3'
Primer1 (for reverse transcription)
   5'-FAM-TGG TCG ATT ACA TGT AC-3'; FAM = fluorescein
Primer2
   5'-TGG TCG ATT ACA TGT ACT GTT TT-3'
Primer3
   5'- AGA TCG GAA GAG CGT CG-3'
Resulting cDNA after reverse transcription: 
5'-FAM-TGG TCG ATT ACA TGT ACT GTT TTC TGT TTG GTT TGT GTT TGT
GTT TTC GTT TGT CGA-N3
Resulting click product: 
5'-FAM-TGG TCG ATT ACA TGT ACT GTT TTC TGT TTG GTT TGT GTT TGT
GTT TTC GTT TGT CGA TAA TGA TAC GGC GAC CAC CGA GAT CTA CAC TCT
TTC CCT ACA CGA CGC TCT TCC GAT CT-3'
 AT = A and T joined via backbone mimic B
Resulting PCR product:

5'-TGG TCG ATT ACA TGT ACT GTT TTC TGT TTG GTT TGT GTT TGT GTT
TTC GTT TGT CGA TAA TGA TAC GGC GAC CAC CGA GAT CTA CAC TCT TTC
CCT ACA CGA CGC TCT TCC GAT CT-3'

### Example 3: Sequence determination of the amplification product

The amplification product was analyzed by a process which determines the sequence of nucleobases in a nucleic acid. Adapter sequences that have been included allow for a hybridization of a complementary oligonucleotide, for immobilization, sequence determination or further amplification.

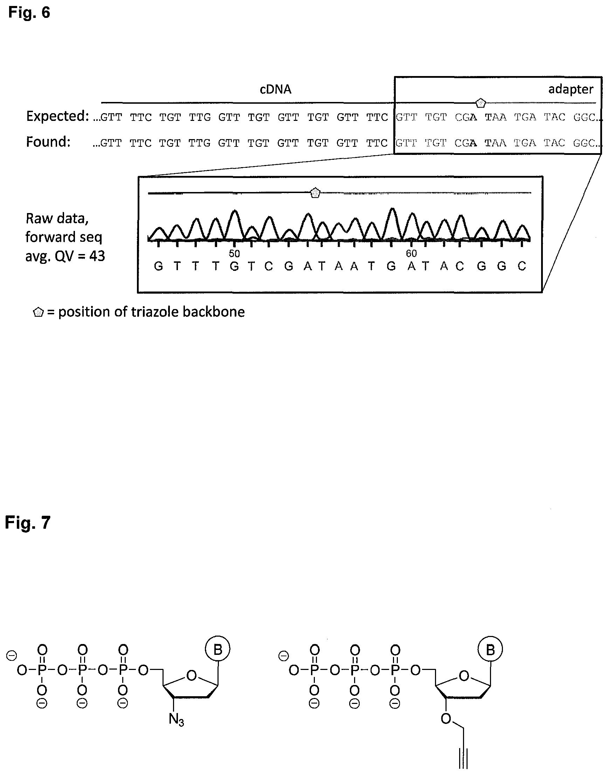

Figure 6 shows the results of Sanger sequencing of one of the amplification products of example 2. It was determined that using the Phusion DNA polymerase, no mutations could be observed at or close by the position of the triazole backbone modification. The result indicates that the click reaction product can be successfully included in a PCR reaction to provide high amounts of PCR products in high efficiency and accuracy.

### Example 4: Low concentration click ligation in the presence and absence of reactor (copper source)

In a 200 µL reaction vial two reactor pellets (600-800 µm, containing elemental copper; sample 1) or no reactor pellets (sample 2) were combined with 12.5 µL reaction mix and incubated at 45 °C for 60 min.

The reaction mix consisted of 800 µM THPTA, 20 mM MgCl₂, 7 µM of alkyne oligo1 and 7 µM of azide oligo1 in dH₂O.

After the incubation the sample was briefly spinned down and the supernatant was transferred to a new vial to stop the reaction. Samples were analyzed on 3% agarose gels (10 x 15 cm) prepared in TAE buffer (20 mM TRIS, 10 mM acetic acid, 0.5 mM EDTA).

Samples were prepared with 20% purple loading dye (NEB), and low molecular weight DNA ladder (25-766 bp, NEB, N3233) was prepared accordingly; usually 0.5 µL marker were used in 5 µL loading volume. Gels were run in TAE buffer applying constant power (10 W, max. 500 V, max. 100 mA) for 60 min. Then, gels were incubated in a freshly prepared 1:10 000 ethidium bromide dilution for 15 min and then destained in dH₂O for 15 min. For visualization a Gel Doc EZ Imager (Bio Rad) was used.

### Oligonucleotides

Alkyne oligo1: 
5'-TAA TGA TAC GGC GAC CAC CGA GAT CTA CAC TCT TTC CCT ACA CGA
CGC TCT TCC GAT CT-3'
T = 5'-alkyne dT 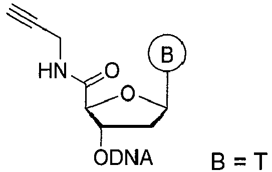
Azide oligo1:

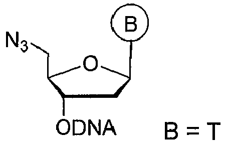

5'-N3-TGG AGT TCG TGA CCG CCG CCG GGA TCA CTC TCG GCA TGG ACG
AGC TGT ACA AGT AAA GC -3'

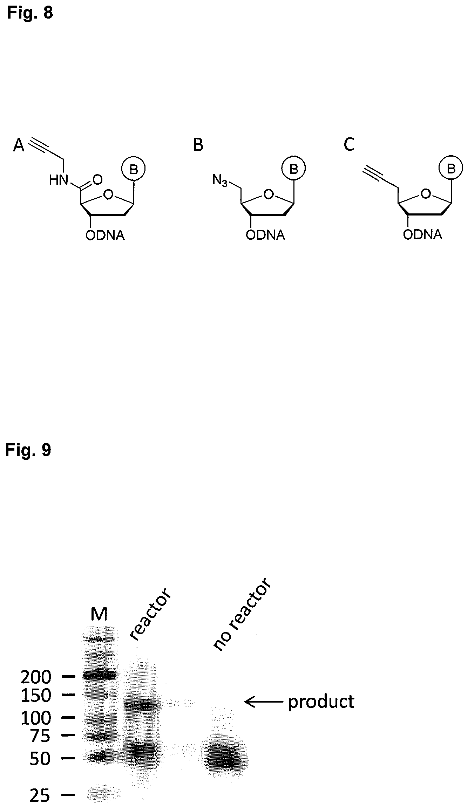

The results of this example are shown in Figure 9. A yield of 36% was obtained after 60 min using the click condition of this example when the reactor was present. When the reactor was omitted no product was observed.

## Claims

1. A method for coupling a first molecule to a second molecule in a click ligation reaction, wherein the first molecule comprises a first click functional group which is an alkyne group, and the second molecule comprises a second click functional group, which is an azide group, the method comprising contacting the first and second molecules in a reaction mixture in the presence of a catalyst, under conditions wherein a click reaction between the first and second molecules occurs, said click reaction being a copper-catalyzed version of a (3+2) cyclo-addition reaction forming a 1,2,3-triazole ring, **characterized in that** the click reaction is performed in the presence of divalent metal cations in the reaction mixture.

2. A method according to claim 1, wherein as divalent metal cations earth alkaline metal cations, preferably Mg²⁺, are present in the click reaction mixture.

3. A method according to claims 1 or 2, wherein the divalent metal cations are contained in the click reaction mixture in an amount of 1 to 200 mmol/l, preferably 5 to 25 mmol/l and most preferably 10 to 20 mmol/l.

4. A method according to anyone of claims 1, 2 or 3, wherein the click reaction mixture comprises an organic solvent, preferably DMSO, and/or a Cu-stabilizing ligand, preferably selected from at least one of Tris(3-hydroxypropyltriazolylmethyl)amine (THPTA), 2-(4-((bis((1-(tert-butyl)-1H-1,2,3-triazol-4-yl)methyl)amino)methyl)-1H-1,2,3-triazol-1-yl)acetic acid (BTTAA), or analogs thereof.

5. A method according to claim 4, wherein of the organic solvent is contained in the click reaction mixture in an amount of 2 to 10%, preferably 4 to 6% and/or the Cu(I)-stabilizing ligand is contained in the click reaction mixture in an amount of 10 to 4000 µmol/l, preferably 500 to 1000 µmol/l.

6. A method according to anyone of claims 1 to 5, wherein the catalyst is a Cu catalyst, preferably a heterogeneous Cu catalyst.

7. A method according to anyone of claims 1 to 6, wherein at least one of the first and second molecules is a biomolecule, preferably selected from nucleosides, nucleotides, nucleic acids, amino acids, peptides, saccharides and lipids and wherein especially preferably both the first and the second molecules are oligonucleotides.

8. A method according to anyone of claims 1 to 7, wherein at least one of the first and second molecules carries a detectable label.

9. An activator composition for use in a click ligation reaction wherein a first molecule comprising a first click functional group, which is an alkyne group, and a second molecule comprising a second click functional group, which is an azide group, are coupled in the presence of a catalyst, preferably a heterogeneous Cu catalyst, said activator mixture comprising divalent metal cations and further an organic solvent and/or a Cu-stabilizing ligand.

10. The activator composition according to claim 9, wherein the divalent metal cations are earth alkaline metal cations, preferably Mg²⁺, and/or the Cu-stabilizing ligand is selected from at least one of Tris(3-hydroxypropyltriazolylmethyl)amine (THPTA), 2-(4-((bis((1-(tert-butyl)-1H-1,2,3-triazol-4-yl)methyl)amino)methyl)-1H-1,2,3-triazol-1-yl)acetic acid (BTTAA), or analogs thereof, and/or the organic solvent is DMSO.

11. The activator composition according to claims 9 or 10, comprising the divalent metal cation in an amount of 1 to 200 mmol/l, preferably 5 to 25 mmol/l, and/or the organic solvent in an amount of 2 to 10%, preferably 4 to 6%, and/or the Cu-stabilizing ligand in an amount of 10 to 4000 µmol/l, preferably 500 to 1000 µmol/l.

12. Click ligation reagent kit, comprising as one component a heterogenous Cu catalyst and as a second component an activator composition according to any one of claims 9 to 11.

13. Click ligation reagent kit according to claim 12, comprising one or more further components of a click reaction, preferably selected from the group consisting of a first molecule comprising a first Click functional group, which is an alkyne group, a second molecule comprising a second Click functional group, which is an azide group, buffers, solvents, enzymes, modified nucleotides, (index) primer and optionally chromatographic material.

14. A device having at least one reaction chamber comprising a heterogeneous Cu catalyst for a click ligation reaction for coupling a first molecule to a second molecule, wherein the first molecule comprises a first click functional group which is an alkyne group, and the second molecule comprises a second click functional group, which is an azide group, and optionally a further solid carrier material, wherein in the at least one reaction chamber of the device divalent metal cations or an activator composition according to 9 to 11 are present.

15. Use of the method according to claims 1 to 8, an activator composition according to claims 9 to 11, a click ligation reagent kit according to claims 12 and 13 or a device according to claim 14, to provide click reaction products, further including subsequent reactions selected from RNA or DNA amplification, RNA or DNA labelling methods and RNA or DNA sequencing methods.
